# EUROPEAN PATENT SPECIFICATION

(11) **EP 3 031 819 B1**
(45) Date of publication and mention of the grant of the patent: **29.11.2017**
(21) Application number: 14835250.3
(22) Date of filing: 05.08.2014
(51) Int. Cl.: C07H 15/10, A61K 8/97, C08B 37/06, A61K 8/68, A61Q 19/00, C07H 1/08, C08B 37/00, A61K 8/73, A61Q 19/02, C07C 231/24, C07C 233/20

(54) **METHOD OF EXTRACTING CERAMIDE AND/OR PECTIN FROM WHOLE APPLES OR APPLE JUICE RESIDUE**
VERFAHREN ZUR EXTRAKTION VON CERMAID UND/ODER PECTIN AUS GANZEN ÄPFELN ODER APFELSAFTRÜCKSTAND
PROCÉDÉ D'EXTRACTION DE CÉRAMIDE ET/OU DE PECTINE À PARTIR DE POMMES ENTIÈRES OU DE RÉSIDU DE JUS DE POMME

(30) Priority: 09.08.2013 JP 2013166275; 31.07.2014 JP 2014156471
(43) Date of publication of application: 15.06.2016
(73) Proprietor: Tsugaru Ringo Ceramides Kabushiki Kaisha, Hirosaki-shi, Aomori 036-8052 (JP)
(72) Inventor: SAKAI, Kenji, Hirosaki-shi Aomori 036-8052 (JP); TANAKA, Kiyoto, Hirosaki-shi Aomori 036-8052 (JP); KAKINAMI, Hiroko, Hirosaki-shi Aomori 036-8052 (JP); ITOKU, Kou, Hirosaki-shi Aomori 036-8052 (JP); KATAYAMA, Hisanobu, Hirosaki-shi Aomori 036-8076 (JP); YAMANO, Yutaka, Hirosaki-shi Aomori 036-8076 (JP); ICHITA, Junji, Aomori-shi Aomori 030-0142 (JP); OSADA, Kyoichi, Kawasaki-shi Kanagawa 214-8571 (JP)
(74) Representative: Dummett Copp LLP
(86) International application number: PCT/JP2014/070613
(87) International publication number: WO 2015/020051

(56) References cited:
- EP-A1- 2 518 088
- WO-A1-2012/148277
- WO-A1-2012/167963
- JP-A- H0 848 702
- JP-A- 2002 253 142
- JP-A- 2005 060 323
- JP-A- 2012 179 034
- WHITAKER,B.D.: 'Analysis of Plant Cerebrosides by C18 and C6 HPLC, Physiology' BIOCHEMISTRY AND MOLECULAR BIOLOGY OF PLANT LIPIDS 1997, pages 143 - 145, XP008182771

## Description

### TECHNICAL FIELD

The present invention relates to a method of extracting useful ingredients: ceramide and pectin from whole apples and/or apple juice residues, and to a method of regenerating or recycling ethanol used for extraction treatment as well.

The term "whole apples" used herein is understood to refer to all as-harvested apples inclusive of not only apple products put on the market for being eaten raw or processing irrespective of full ripeness or unripeness, but also apple products that retain original form like as-yet-not-processed (extracted) apples. That is, the "whole apples" refer to all as-harvested apples that retain original form like fallen apples, unripe apples, and apples excluded out of a sorting process. In other words, the "whole apples" refer to all apples that are not subjected whatsoever to any special processing resulting in deformation after harvesting.

### BACKGROUN OF THE INVENTION

In general, apple fruits contain not only pectin and other useful ingredients such as dietary fibers in abundance but also a variety of useful ingredients such as ceramide, polyphenols and potassium, and there are some reports about various effects of these ingredients working on the maintenance and improvement of health.

Incidentally, apple juice residues occur in large amounts upon production of apple juices from apple fruits with the result that composite ingredients occurring from those residues give rise to many cumbersome problems such as air pollution or environmental pollution like foul odors and putrid smells. In the seasons of summer and mild climate in particular, these residual ingredients rot more rapidly, so disposal of apple waste surfaces as an urgent issue to be addressed from the standpoint of pollution control too. It is also desired to take full advantage of apple waste, and there is a mounting demand for the development of a method of extracting yet unavailable useful ingredients contained in apple juice residues.

### PRIOR ARTS

### Patent Publications

Patent Publication 1: JP(A) 2005-60323
Patent Publication 2: JP(A) 2011-109956

There are some prior art publications that would appear to be pertinent to the present invention, as described below.

Patent Publication 1 relates generally to the provision of an allergen-free ceramide capable of being well mixed with other ingredients. However, Patent Publication 1 teaches away from the present invention: it discloses or suggests nothing about both the "whole apples" and the extraction of pectin.

Patent Publication 2 relates generally to the use of ethanol for sugar production. However, this publication discloses or suggests nothing about the extraction and refinement of pectin and ceramide derived from apples either.

### Non-Patent Publications

### [Non-Patent Publication 1]

"Content of Functional Lipid Ceramide in Agricultural Products and Processed Byproducts Thereof' by the distribution system research team of the upland farming research division, Hokkaido Agricultural Research Center
1. Non-patent Publication 1 discloses a method of extracting ceramide contained in agricultural products and processed byproducts thereof. Set out below are the excerpts.

### [Non-Patent Publication 1]

"Content of Functional Lipid Ceramide in Agricultural Products and Processed Byproducts Thereof' by the distribution system research team of the upland farming research division, Hokkaido Agricultural Research Center

In Non-Patent Publication 1 there are "Summary" and the following reports concerning the subject matters.

Ceramide that is a sort of glycolipid is used for foodstuff and cosmetic raw materials because of having effects on moisturization and whitening of the skin. "To search for materials suitable for low-cost production ..., the contents of ceramide in various agricultural products and processed byproducts thereof were investigated."
2. Testing Method (concerning the contents of ceramide in agricultural products and processed byproducts thereof)

In what follows, the testing method of Non-Patent Publication 1 will be explained.

### (1) Extraction and Quantification of Ceramide

A ceramide fraction extracted from various dried and ground samples with a mixed chloroform-methanol solution was developed in thin-layer chromatography to estimate the concentrations of ceramide and sterol glycoside from a spot concentration as compared with a standard substance.

### (2) Analysis of the Ceramide Components

The constitutive sphingoid base and fatty acid of ceramide were analyzed by means of gas chromatography/mass spectrometry.

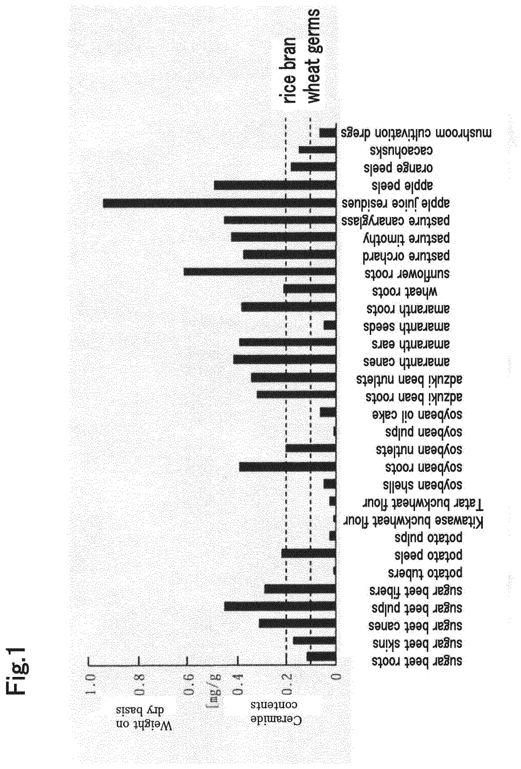

### (3) The results of the aforesaid test are presented in graphical form in Fig. 1 as per attached "Contents of Ceramide in Agricultural Products and Processed Byproducts Thereof".

### (Test Results and Discussions)

(1) There is ceramide present in all the samples under test, shown in Fig. 1. The content has a distribution of 0.01 to 0.94 mg/g, among which the apple juice residue has the highest content of ceramide. Noteworthy in Fig. 1 is that it is only apple peels and apple juice residues that contain ceramide in a content of 0.5 mg/g or greater except for sunflower roots. The apple juice residues in particular have a ceramide content of 0.94 mg/g, almost twice as high as that of other samples under test.
(2) In an existing general ceramide production process, sterol glycoside in plants renders high-purity refinement of ceramide difficult, and the samples under test contain it in an amount of 0.01 to 0.87 mg/g.
(3) The apple juice residues have a ceramide/sterol glycoside ratio of 1.09 or, in another parlance, they have the lowest proportion of sterol glycoside relative to ceramide, facilitating high-purity refinement of ceramide. Other samples have a ceramide/sterol glycoside ratio of the order of 0.02 to 0.06.
(4) A main constitutive ceramide species of the apple juice residues is 2-hydroxypalmitic acid as a fatty acid, and 4-hydroxy-cis-8-sphingenine as a sphingoid base; it is expected to have a composition similar to that of a common plant ceramide and, hence, have a similar functionality. Note here that the chemical structure of apple ceramide in Non-Patent Publication 1 is shown in Figure 2 attached hereto.

### (Discussions)

Non-Patent Publication 1 shown in Fig. 1 attached hereto demonstrates that among the samples under test used therein, the apple juice residue has the highest ceramide content as compared with the rest.

### SUMMARY OF THE INVENTION

### OBJECTS OF THE INVENTION

One object of the invention is to provide a method of extracting useful ingredients: ceramide and/or pectin from whole apples and/or apple juice residues, and another object of the invention is to provide a method of regenerating or recycling ethanol used for the extraction as well.

### HOW TO ACCOMPLISH THE OBJECTS

The present invention provides a method of extracting useful ingredients: ceramide and/or pectin from whole apples and/or apple juice residues, and provides a method of regenerating or recycling ethanol used for the extraction as well.

More specifically, the present invention is embodied as follows.

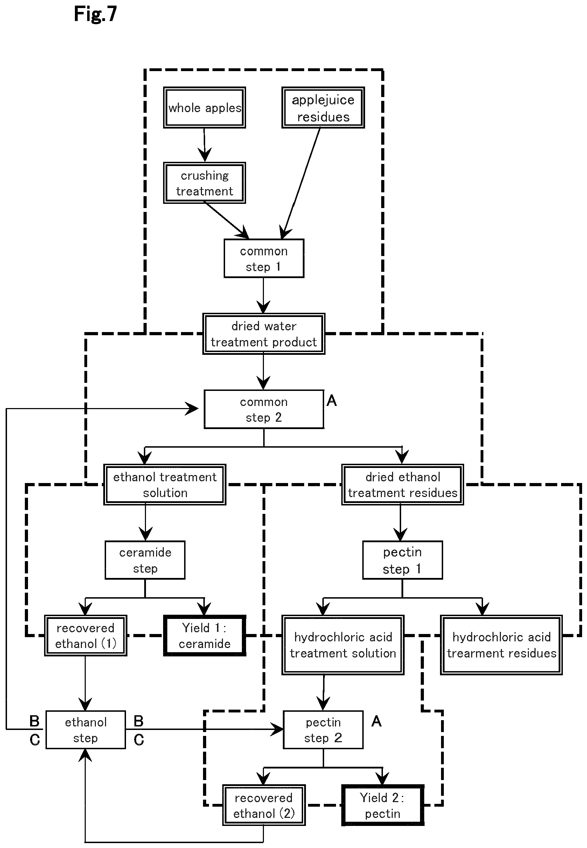

According to the invention, the method of extracting ceramide and/or pectin, and the method of recovering, regenerating and recycling ethanol comprises:
(a) a step of treating whole apples and/or apple juice residues with water and drying the residues (common step 1), treating the residue with ethanol and drying the residues to recover an ethanol treatment solution containing ceramide and an ethanol treatment residue containing pectin, and drying the ethanol treatment residue to obtain a dried ethanol treatment residue (common step 2) (Figure 7),
(b) a step of refining ceramide from the ethanol treatment solution (ceramide step comprising concentrating treatment, ethanol recovery treatment and refining treatment),
(c) a step of extracting pectin from the dried ethanol treatment residue for refinement (pectin steps 1 and 2 comprising hydrochloric acid treatment, concentrating treatment, separation of pectin and drying treatment), and
(d) a step of recovering ethanol used in steps (b) and (c) for regeneration and/or recycling (ethanol step).

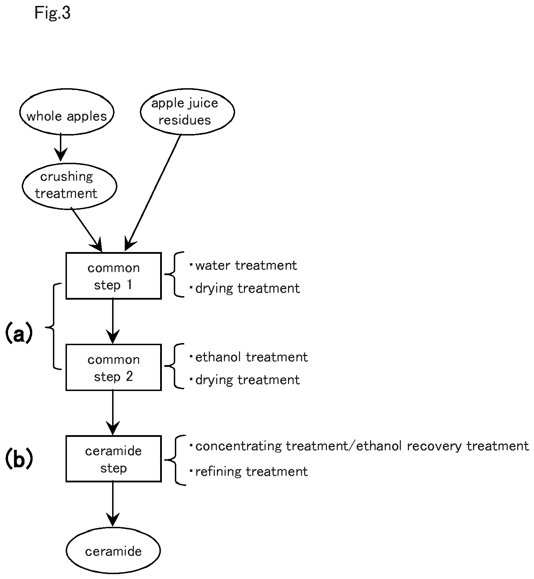

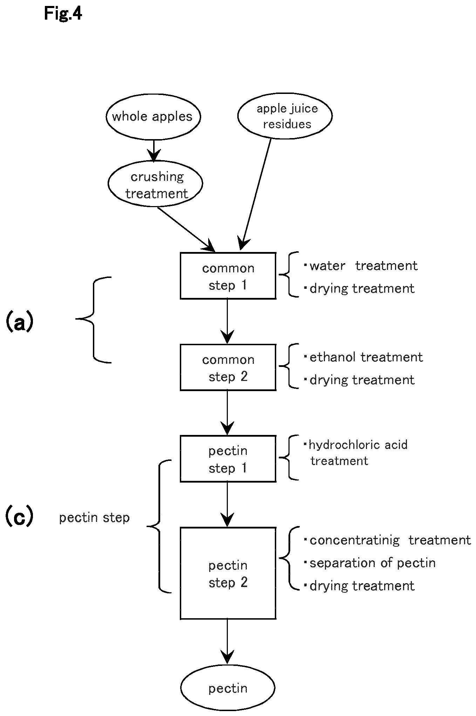

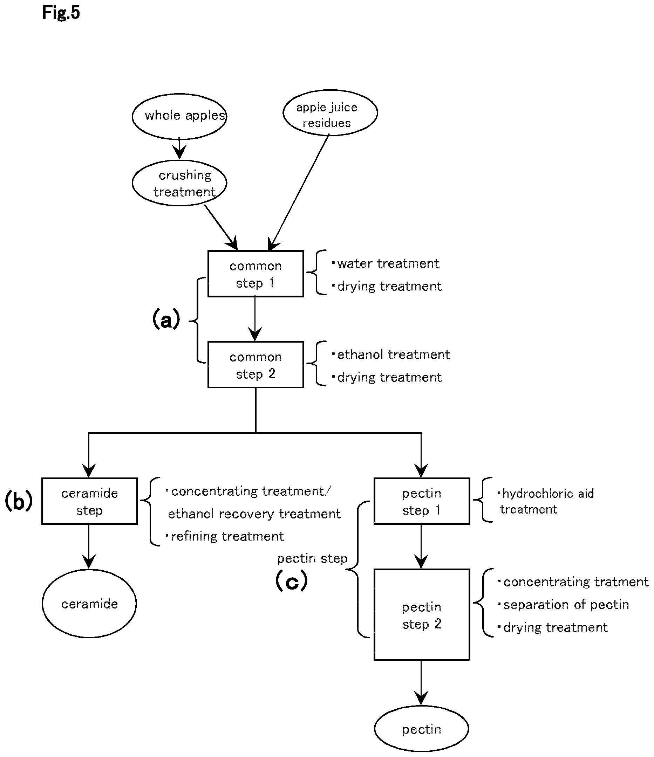

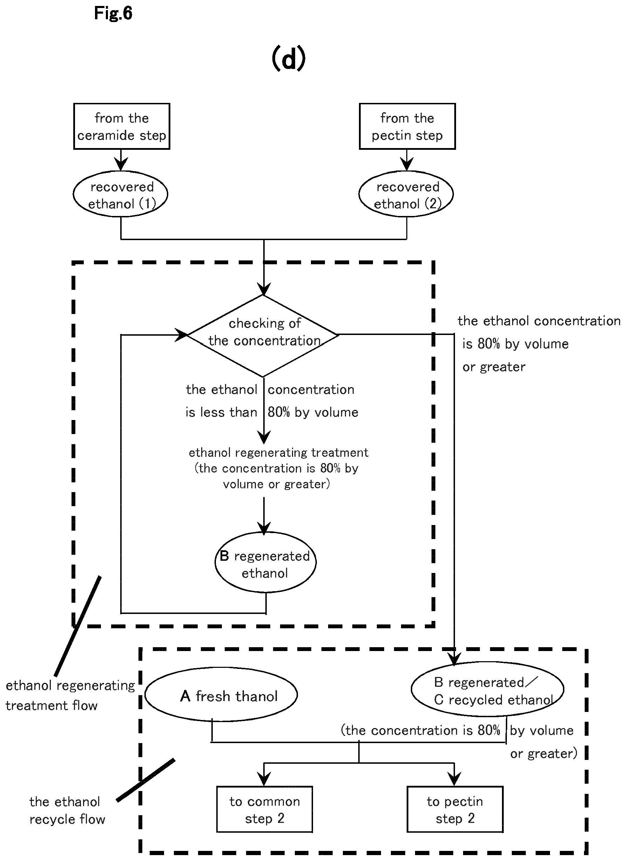

In what follows, step (a) will be referred to as common step 1, 2 (Figures 3, 4 and 5); step (b) as the ceramide step (Figures 3 and 5); step (c) as the pectin step (pectin step 1, 2: Figures 4 and 5); and step (d) as the ethanol step (Figures 6 and 7).

Prior to common step 1, water treatment and other steps, the whole apples are crushed, mashed and optionally disintegrated to pieces to improve the treatment efficiency in the following common step 1, 2, etc. To this end, the whole apples may be crushed and mashed to pieces (crushing of individual apples) and then mixed or otherwise processed with the "apple juice residues" to allow the "whole apples" to have an average water content substantially equal to that of the "apple juice residues". In other words, the "whole apples" refer to apples prior to juice extraction treatment while the "juice residues" refer to apples after juice extraction treatment.

The juice residues, because of having already been crushed to pieces, may directly or immediately be delivered to common step 1 and the following steps (water treatment, etc.).

### (Order of the ceramide and pectin steps)

In the invention described herein, the ceramide step, and the pectin step (comprising a combination of pectin step 1 and pectin step 2 carried out in this order) should be carried out in this order. That is, upon completion of the aforesaid common step 2, the ethanol treatment solution necessary for the ceramide step and the dried ethanol treatment residue necessary for the pectin step are separately obtained at the same time.

It is here to be noted that common steps 1 and 2 also serve as common pretreatments prior to both the ceramide step and the pectin step. Although the ethanol treatment solution and the dried ethanol treatment residue are respectively obtained as the result of common step 2, it is understood that the subsequent ceramide and pectin steps make separate, independent progress. As a result, ceramide is yielded in the ceramide step and pectin is yielded in the pectin step (Figure 7).

In other words, the ethanol treatment solution and the dried ethanol treatment residue are obtained separately at the same time in this stage (Figure 7). Thereafter, the ceramide step or the pectin step may be carried out separately in any desired order: the former may first be carried out and the latter is then carried out, and vice versa. As mentioned above, in the stage through common step 2, the ethanol treatment solution and the dried treatment residue are obtained at the same time (Figure 7). Thereafter, the ceramide step and the pectin step may be carried out either independently or concurrently.

In another parlance, the ceramide step and the pectin step (comprising a combination of pectin step 1 and pectin step 2 carried out in this order) may be carried out independently, in stages, or individually. This means that the ceramide step and the pectin step may take place concurrently or reversibly in order.

The "whole apples" used herein is understood to refer to all as-harvested apple products inclusive of not only apple products put on the market for being eaten raw or processing, but also apple products that retain apple form like as-yet-not-processed apples. That is, the "whole apples" refer to all as-harvested apples that retain apple form like fallen apples, unripe apples, and apples excluded out of a sorting process.

The "apple juice residues" used herein are understood to refer to a solid part separated from a liquid part by subjecting fruits of deciduous high trees belonging to Malus such as Rosaceae and Maloideae to a juice-extraction process known to those skilled in the art, for instance, a juice-extraction process comprising crushing, mashing and squeezing or, alternatively, simply crushed and mashed fruits. By way of example but not by way of limitation, apple fruits are crushed by a hammer crusher to a size of about 5 to 30 mm, and then squeezed at a pressure of about 5 kg/cm² to 20 kg/cm². It is to be noted, however, that apple fruits vary largely in state with kinds; the purpose of this step is to carry out crushing or squeezing so as to improve the efficiency of the following steps. There is no particular limitation on the degree of crushing or squeezing. The crushing or mashing treatment may optionally be followed by disintegrating treatment.

It is also to be understood that the "apple juice residues" used herein include not just a "juice residue" obtained by juice-extraction treatment in apple juice-extraction plants but also a so-called "apple processing byproduct" occurring in various processes in juice-extraction plants, juice production plants, processing plants or the like inclusive of peels or cores left behind after cutting.

It is further to be noted that the "ceramide" described herein refers to "cerebroside" comprising ceramide to which glucose or galactose is bonded, rather than a compound composed of sphingosine and a fatty acid bonded by way of an amide bond. Although what is derived from plants such as apples and called ceramide is present in this cerebroside state, yet the name ceramide stick generally in the art. This is the reason that that name is used herein. That is, the "ceramide" used herein includes cerebroside comprising glucose or galactose bonded to ceramide that is a compound composed of sphingosine and a fatty acid by way of an amide bond.

### ADVANTAGES OF THE INVENTION

According to the method of the invention for extracting ceramide and pectin from whole apples and/or apple juice residues, whole apples and/or apple juice residues, most of which are thrown away as industrial wastes, can be utilized for foodstuff materials and cosmetic materials. The invention described herein generally has such advantages as mentioned below.

### (Advantage 1)

According to the invention, unused useful ingredients contained in whole apples or apple juice residues are obtained in the form of ceramide and pectin. Especially in the initial stage of the overall process of the invention (i.e., the initial steps of Figures 3 and 4), the water treatment (common step 1: water treatment and drying treatment) and the ethanol treatment (common step 2: ethanol treatment and drying treatment) are used as a common step for effective removal of water-soluble ingredients contained in whole apples or apple juice residues.

### (Advantage 2)

In the invention described herein, the aforesaid ceramide and pectin steps may be carried out concurrently or reversibly in order ([0013], [0014]) for individual extraction of ceramide or pectin. The aforesaid concurrency or reversibility in order ([0013], [0014]) allows for prevention of overlapping of the steps or processes, resulting in avoidance of double investment. This makes it possible to extract and refine useful ingredients derived from apples in a more effective way in a less space.

### (Advantage 3)

In the invention described herein, both the whole apples and the juice residues may be treated for prevention of overlapping of installations for treating two different matters and shortening of the overall treatment time.

In common step 1, the effects of the water treatment on removal of water-soluble ingredients from whole apples and/or apple juice residues are set out in Table 1.

**Table 1**

| Effects of the water treatment on removal of water-soluble ingredients from apple juice residues (Water treatment effect A) | | |
|---|---|---|
| | Juice residues before drying | Weight of residues after drying |
| No water treatment | 33.0kg (100%) | 6.0kg (18.2%) |
| Water treatment | 33.0kg (100%) | 3.6kg (10.9%) |

### (Conditions for acquiring the data of Table 1)

Set out below are the conditions for acquiring the data of Table 1.

Two sets of apple juice residues, 33.0 kg per each, are provided. One set is placed and dried in a warm air type drier held at a drying temperature of 80°C (with no water treatment). Another set is placed in a vessel having a volume of 200 liters, and added with 100 liters of water. After the addition of water, stirring is carried out for 30 minutes until the whole is put in a uniform state. After stirring, the water solution is filtered through a filtering strainer having a mesh diameter of 180 µm to separate off the juice residues. The separated juice residue is transferred in the aforesaid vessel, and a cycle of addition of water→stirring→filtration is repeated an additional three times. The juice residue separated off by the final filtration is dried in a warm air type dryer held at a drying temperature of 80°C (with water treatment). In either case, the time needed for drying is about 16 hours, and after the completion of drying, the juice reside is taken out from the dryer and weighed. The weight of the juice reside measured after drying is shown in Table 1.

As set out in Table 1, the weight of the juice residue after drying was 3.6 kg with the water treatment applied whereas the weight was 6.0 kg without the water treatment. This means that the weight of the residue after drying with the water treatment applied decreases relatively about 40%.

The "water-soluble ingredients" described herein refer to such ingredients as sucrose, fructose, glucose and malic acid except for the end substances of the invention: ceramide and pectin. Note here that the "water-soluble ingredients" referred to herein do not include any "water-soluble dietary fiber".

It is here to be understood that the crushing, mashing or disintegrating treatment allows for ready and smooth water treatment, etc. in the next common step 1. That is, the crushing, mashing or disintegrating treatment of whole apples ensures that the whole apples are put in much the same state as is the case with the apple juice residues. As a result, the whole apples could be treated by the inventive process in common step 1 etc. even alone or in combination with the apple juice residues. In other words, the "apple juice residues" are defined as an apple residue after juice extraction while the "whole apples" are defined as individual apples before juice extraction.

### (Relations of the presence or absence of the water treatment to the concentration of recovered ethanol)

In the invention described herein, the presence or absence of the water treatment has such relations to the concentration of recovered ethanol as shown in Table 2.

**Table 2**

| The presence or absence of the water treatment and the concentration of recovered ethanol (Water Treatment Effect B) | | |
|---|---|---|
| | Concentration of ethanol used for extraction | Concentration of ethanol recovered upon refinement of ceramide |
| No water treatment | 99.5% | 93.0% |
| Water treatment | 99.5% | 99.0% |

### (Conditions for acquiring the data of Table 2)

Set out below are the conditions for acquiring the data of Table 2.

The juice residues dried with the water treatment applied and the apple juice residues dried with no water treatment applied are each provided in an amount of 500 g. Each apple juice residue is poured in a beaker having a volume of 5 liters, and 2.5 liters of ethanol having a concentration of 99.5% is added to the beaker. Stirring is carried for 24 hours in such a way that the whole is placed in a uniform state. After stirring, the juice residue and the ethanol solution are separated by suction filtration using a filter paper. Using a rotary evaporator, the ethanol solution is concentrated until its volume is reduced down to 1/10 to recover ethanol together with a concentrate. The specific gravity of the recovered ethanol is measured by a glass hydrometer, and the concentration of the recovered ethanol is found out using a correlation table relating to the value of the found specific gravity, specific gravity and ethanol concentration. The respective concentrations of the recovered ethanol are shown in Table 2.

As can be seen from Table 2, even when the water treatment is applied, there is no or little decrease in the concentration of the recovered ethanol. To put it another way, the water treatment results in only a decrease in the concentration of the recovered ethanol from 99.5% down to 99.0%.

That is, when the water treatment is applied, the recovered ethanol is less likely to decrease as compared when no water treatment is applied. This indicates that the ingredients likely to hold moisture (the "water-soluble ingredients" referred to in Table 1) are removed off so much so that the efficiency of drying is improved resulting in a decrease in the remaining water content (Water Treatment Effect A).

As a result of the water treatment, the concentration of the recovered ethanol is less likely to decrease. This means that the recovered ethanol may be recycled (more readily than that obtained with no water treatment applied) because its concentration (99.0%) is almost equivalent to the concentration (99.5%) of ethanol before used for the ethanol treatment, as appreciated from a concentration difference: 93.0% and 99.0% in Table 2 (Water Treatment Effect B).

### (Advantages of the invention as recited in the respective claims)

According to claim 1, it is possible to extract ceramide from whole apples and/or apple juice residues.

According to claim 2, it is possible to recycle ethanol used for extraction of ceramide from whole apples and/or apple juice residues.

According to claim 3, it is possible to recycle ethanol used for extraction of ceramide from whole apples and/or apple juice residues after its regeneration.

According to claim 4, it is possible to extract pectin from whole apples and/or apple juice residues.

According to claim 5, it is possible to recycle ethanol used for extraction of pectin from whole apples and/or apple juice residues.

According to claim 6, it is possible to recycle ethanol used for extraction of pectin from whole apples and/or apple juice residues after its regeneration.

According to claim 7, it is possible to independently extract and refine ceramide and pectin from whole apples and/or apple juice residues.

According to claim 8, it is possible to concurrently extract and refine ceramide and pectin from whole apples and/or apple juice residues.

According to claim 9, it is possible to extract and refine ceramide and pectin from a mixture of whole apples and apple juice residues individually, in stages, or concurrently.

According to claim 10, it is possible to use at least two of fresh ethanol, regenerated ethanol and recycling ethanol in a mixed form.

### BRIEF EXPLANATION OF THE DRAWINGS

Fig. 1 is indicative of the contents of functional lipid ceramide in agricultural products and their processed byproducts.

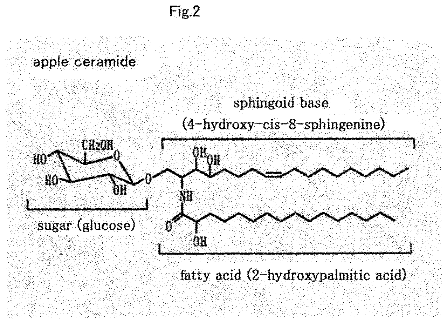

Fig. 2 is representative of the chemical structure of apple ceramide.
Fig. 3 is illustrative of the steps of extracting ceramide from whole apples and/or apple juice residues.
Fig. 4 is illustrative of the steps of extracting pectin from whole apples and/or apple juice residues.
Fig. 5 is illustrative of the steps of extracting ceramide and pectin from whole apples and/or apple juice residues.
Fig. 6 is illustrative of the steps capable of regenerating and recycling the spent ethanol.
Fig. 7 is generally illustrative of the overall extraction flow for ceramide and pectin according to the invention.
Fig. 8 is representative of material balance in the overall extraction of ceramide and pectin from whole apples and apple juice residues.

### MODE FOR CARRYING OUT THE INVENTION

The mode for carrying out the invention comprises the following respective processes alone or in combination.
[1] A method of extracting ceramide from whole apples and/or apple juice residues, characterized by extraction and refinement of ceramide comprising a common step 1 of applying water treatment to whole apples and/or apple juice residues (water treatment/drying treatment), a common step 2 of applying ethanol treatment (ethanol treatment/drying treatment), and a ceramide step (concentrating treatment, ethanol recovery treatment and refining treatment).
[2] A ceramide extraction method, wherein the ethanol used for said extraction and refinement of ceramide is recovered and regenerated into a recyclable state and then recycled (ethanol recovery, checking of ethanol concentration, ethanol regeneration, checking of ethanol concentration, and recycling of ethanol).
[3] A ceramide extraction method, comprising recovery and recycling of the ethanol used for said extraction and refinement of ceramide (ethanol recovery, checking of ethanol concentration, and recycling of ethanol).
[4] A method of extracting pectin from whole apples and/or apple juice residue, characterized by comprising a common step 1 (water treatment/drying treatment) of applying water treatment to whole apples and/or apple juice residue, a common step 2 including ethanol treatment (ethanol treatment/drying treatment), a pectin step 1 of applying hydrochloric acid treatment, and a pectin step 2 including concentrating treatment, separation of pectin and drying treatment.
[5] A pectin extraction method, wherein the ethanol used for said extraction and refinement of pectin is recovered and regenerated into a recyclable state and then recycled (recovery of ethanol, checking of ethanol concentration, regeneration of ethanol, checking of ethanol concentration, and recycling of ethanol).
[6] A pectin extraction method, wherein the ethanol used for said extraction and refinement of pectin is recovered into a recyclable state (recovery of ethanol, checking of ethanol concentration, and recycling of ethanol).
[7] A method of extracting ceramide and pectin from whole apples and/or apple juice residues comprising a common step 1 for the whole apples and/or apple juice residue, a common step 2, a ceramide step (concentrating treatment, ethanol recovery treatment, and ethanol refining treatment), a pectin step 1 comprising hydrochloric acid treatment, and a pectin step 2 comprising concentrating treatment, separation of pectin and drying treatment, characterized in that extraction and refinement of ceramide and pectin from the whole apples and/or apple juice residues are independently carried out.
[8] A method of extracting ceramide and pectin from whole apples and/or apple juice residues, comprising a common step 1 for whole apples and/or apple juice residues, a common step 2, a ceramide step (concentrating treatment, ethanol recovery treatment, and refining treatment), a pectin step 1 comprising hydrochloric acid treatment, and a pectin step 2 comprising concentrating treatment, separation of pectin and drying treatment, characterized in that extraction and refinement of ceramide and pectin from whole apples and/or apple juice residues are concurrently carried out.
[9] A method of extracting ceramide and pectin from whole apples and/or apple juice residues comprising a process including a common step 1 to which a mixture of whole apples and juice residues is fed, a common step 2, a ceramide step, a pectin step 1, and a pectin step 2, characterized in that extraction and refinement of ceramide and pectin from whole apples and/or apple juice residues are carried out individually and in stages.
[10] A method of extracting ceramide and pectin from whole apples and/or apple juice residues comprising a process including a common step 1 to which a mixture of whole apples and juice residues is fed, a common step 2, a ceramide step, a pectin step 1, and a pectin step 2, characterized in that extraction and refinement of ceramide and pectin from whole apples and/or apple juice residues are concurrently carried out.
[11] A method of using ethanol, characterized in that in a method of extracting ceramide and/or pectin from whole apples and/or apple juice residues, at least two of fresh ethanol, regenerated ethanol, and recyclable ethanol are mixed for use.

### Example 1

In what follows, the respective steps or constitutive elements of the invention will be explained; Example 1 of the method of extracting ceramide and pectin will be explained with reference to Figures 3 to 7 in this order.

Common step 1 comprises water treatment, and drying treatment. Note here that the water treatment may be left out when low purity is allowable for the finally obtained products: ceramide and pectin.

### (Common Step 1) (Figures 3, 4, 5 and 7)

### Common Step 1-1 (for water treatment/drying treatment)

Whole apples and/or apple juice residues may be provided in such a way as to have a weight or volume ratio of approximately 1:4 to water. For instance, 4 liters of water are added to 1 kg of apple juice residues and stirred for about 5 minutes. Industrially usable water such as tap water or underground water may be used for this stirring treatment, and stirring may be carried out by stirring means such as a bladed stirrer. Any other known stirring methods may be used provided that water is well mixed with water juice residues.

After the completion of stirring, filtration may be carried out using filtration means such as a filtering strainer or filter. The filtering strainer or filter may have such a pore diameter as to drain the juice residues, although an average pore diameter of less than 200 µm is preferred.

In the presence of water, stirring and filtration (or one set of stirring and filtration) may be repeated alternately about several times, preferably about four times to drain the juice residues thoroughly, and they are then delivered out to the drying treatment step.

### Common Step 1-2 (Drying Treatment)

After the apple juice residues obtained by the water treatment are thoroughly drained, they are dried in drying means such a warm air type dryer until their water content decreases down to about 10% or less. Here, as the juice residues are well dried until their water content is down to about 10% or less, it prevents the ethanol concentration from decreasing due to the water content in the ethanol treatment in the next common step 2.

Referring here to the water treatment of the "whole apples", the purpose of removing ingredients (viz., the water-soluble ingredients) other than the end ingredients holds true for the juice residues; that is, there is no particular disadvantage with a mixture of them with the "juice residues". The reason is that, in the invention described here, the efficiency of extraction of the end ingredients: ceramide and pectin is brought up by removal of ingredients (viz., the water-soluble ingredients) other than the end ingredients by the water treatment.

### (Common Step 2) (Figures 3, 4, 5 and 7)

Common step 2 comprises ethanol treatment (2-1) and drying treatment (2-2).

### (Ethanol treatment in common step 2-1)

The weight or volume ratio of ethanol to the water treated and dried product is set at about 1:4. For instance, 4 liters of ethanol are added to 1 kg of the water treated and dried product and stirred over about 2 hours. The ethanol used here has preferably a concentration of 80% by volume or greater, because in an ethanol concentration less than 80% by volume, ceramide is less likely to be dissolved in ethanol, resulting in a drop of extraction efficiency. As is the case with common step 1, stirring may be carried out by stirring means such as a bladed stirrer.

After the completion of stirring, filtration is carried out using filtration means such as a filtering strainer or filter. Preferably in this case, the filtration means such as a filtering strainer or filter have a pore diameter of 200 µm or less, because there is no sufficient filtration effect obtained in a pore diameter of greater than 200 µm. Note here that the separation of ethanol from the juice residues may also be carried out by known methods such as centrifugal filtration.

### (Common step 2-2: Drying Treatment)

The residues obtained by the ethanol treatment are then dried by drying means such as a warm air type dryer until their water content is down to about 10%. Note here that depending on drying environments or the like, air drying may be selected because ethanol is relatively volatile.

### (Ceramide Step) (Figures 3, 5 and 7)

The ceramide step comprises concentrating treatment/ethanol recovery treatment, and refining treatment.

### (Ceramide step) - (Concentrating Treatment/Ethanol Recovery Treatment)

While the ethanol treatment solution is concentrated, the ethanol used for the ethanol treatment is recovered. Concentration may be carried out by concentrating means such as a rotary evaporator. Ethanol may be removed out from the ethanol treatment solution for concentration, and the removed ethanol may be recovered by recovery means such as the aforesaid rotary evaporator. Concentration is preferably carried out by heating at a temperature lower than the boiling point under normal or reduced pressure. The equipment necessary for reduced pressure includes a vacuum means such as a circulation type aspirator. Any other vacuum methods may also be used provided that the necessary degree of a reduced pressure (about 0.1 atm.) or vacuum is maintainable.

### (Ceramide Step) - (Refining Treatment)

The concentrated ethanol treatment solution is refined to obtain ceramide. For refinement, ingredients other than ceramide may be removed by chromatography using activated clay, active charcoal, silica gel, alumina, diatomaceous earth, synthesized adsorbents, ion exchange resin or the like, adsorption, decomposition, precipitation, filtration, dissolution, distillation or the like.

To separate the concentrated ethanol treatment solution into ceramide and other ingredients or, alternatively, a ceramide-containing fraction and a ceramide-free fraction (note here that a fraction is obtained by separating a substance comprising a plurality of mixed ingredients into them), activated clay, active charcoal, silica gel, alumina, diatomaceous earth, synthesized adsorbents, ion exchange resin or the like used in the aforesaid refining process may optionally be combined together for separation.

### (Pectin Step) (Figures 4, 5 and 7)

Pectin step 1 comprises hydrochloric acid treatment (Figures 4, 5 and 7).

### (Pectin Step 1)

### (Hydrochloric Acid Treatment)

The dried ethanol treatment residues obtained by drying after the ethanol treatment are provided in such a way as to have a weight or volume ratio of about 1:90 relative to hydrochloric acid. For instance, 90 liters of hydrochloric acid are added to 1 kg of the dried ethanol treatment residues, and stirring is carried out for about 30 minutes while they are heated to a temperature of about 75°C to about 85°C. The concentration of hydrochloric acid used here is preferably 0.01 N to 0.2 N. As is the case with common step 1, stirring may be carried out by stirring means such as a bladed stirrer. After the completion of stirring, filtration is carried out using filtrating means such as a filtering strainer or filter. The filtrating means used here, such as a filtering strainer or filter, have preferably a pore diameter of 200 µm or less.

Alternatively, centrifugal filtration or the like may be used as the filtration means provided that there can be a separation of the dried ethanol treatment residues from hydrochloric acid.

### (Pectin Step 2)

Pectin step 2 comprises concentrating treatment, separation of pectin, and drying treatment (Figures 4, 5 and 7).

### (Concentrating Treatment)

The hydrochloric acid treatment solution is concentrated. As is the case with the ceramide step, concentration may be carried out by concentrating means such as a rotary evaporator.

### (Separation of Pectin)

Ethanol is added to the concentrated hydrochloric acid solution in a volume ratio of about 1:2 to about 1:5, and the resulting solution is stirred over about 30 minutes for precipitation of gel-like pectin. The concentration of ethanol used here is preferably 80% by volume or greater. As is the case with common step 1, stirring may be carried out by stirring means such as a bladed stirrer.

After the completion of stirring, the settled-down gel-like pectin is separated off using filtrating means such as a filtering strainer or filter. While the filtrating means such as a filtering strainer or filter have preferably a pore diameter of 200 µm or less, it is understood that there is no limitation on it provided that there is the capability of having a function of separating the settled-down gel-like pectin off from the liquid part.

Much the same amount of ethanol as mentioned above is added to the obtained gel-like pectin; that is, there is ethanol added to it in an amount of about 1:2 to about 1:5 by volume that is the amount of ethanol added to the aforesaid concentrated hydrochloric acid treatment solution for an about 5-minute stirring. After the completion of stirring, the settled-down gel-like pectin is separated off using filtrating means such as a filtering strainer or filter. The ethanol, stirring and filtration used to this end may be pursuant to what has been described herein.

### (Drying Treatment)

The gel-like pectin separated off from the hydrochloric acid treatment solution is dried using drying means such as a freeze dryer or a spray dryer until its water content is down to about 10% or less. The pectin obtained by drying may be ground and sieved out.

### (Ethanol Step) (Figures 6 and 7)

To recover and recycle the ethanol used once in common step 2 in the ceramide step and the ethanol used in pectin step 2, the concentration of ethanol must be 80% by volume or greater. Note here that in a concentration of 80% by volume or less, the ethanol must be regenerated to bring its concentration up.

How to regenerate ethanol is now explained mainly with reference to the ethanol regeneration treatment flow of Figure 6.

### (Regeneration of Ethanol)

In the aforesaid pectin step 2, the ethanol used once and then recovered suffers from a decrease of concentration by incorporation of water in the step of using it. Therefore, the concentration of ethanol is first checked to recycle the ethanol used once and then recovered. When the concentration of ethanol is found to been less than 80% by volume, that concentration is brought up to 80% by volume or greater by distillation or the like. In what follows, the wording "the concentration of ethanol is brought up to 80% by volume or greater to place it in a recyclable state" is called the "regeneration of ethanol". The ethanol regenerated after recovery into a recyclable state is here defined as the "regenerated ethanol" (B), whereas the ethanol recyclable immediately after recovery without any regeneration is called the "recyclable ethanol" (C).

To put it another way, the "regeneration of ethanol" described herein implies that the concentration of ethanol is brought up to 80% by volume or greater into a recyclable state. With this in mind, the step of treating the ethanol recovered in the ceramide step and pectin step 2 comprises a two-stage process wherein the ethanol is "regenerated" through "checking of the concentration of ethanol" into a recyclable state and then used or, alternatively, a one-stage process wherein the ethanol is "recycled" immediately through "checking of the concentration of ethanol".

In the aforesaid pectin step 2, the ethanol used once and then recovered suffers from a lowering of concentration by incorporation of water in the step of using it. In the recovery and recycling of ethanol used once, the concentration of ethanol is first checked. When the concentration of ethanol is found to be down to less than 80% by volume, the concentration is brought up to 80% by volume or greater by a process such as distillation. In what follows, "the concentration of ethanol is brought up to 80% by volume or greater into a recyclable state" is called the "regeneration of ethanol".

### (Checking of the Concentration of Ethanol)

In the ethanol treatment step of using ethanol other than fresh one, the concentration of ethanol is first checked. How to check the concentration of ethanol includes known methods such as a method of finding concentration from density by calculation, and a method of finding concentration from boiling points by calculation.

When the concentration of ethanol is found to be 80% by volume or greater, the ethanol is immediately recycled. When the concentration is found to be less than 80% by volume, the ethanol is regenerated before recycling.

### (Regenerating treatment of ethanol when the concentration of ethanol is found to be less than 80% by volume: Figure 6)

Ethanol having an ethanol concentration of less than 80% by volume is brought up to a concentration of 80% by volume or greater by distillation. For instance, distillation may be carried out at 50°C to 150°C under atmospheric pressure.

Alternatively, a similar process such as distillation under reduced pressure may be used too. The time necessary for distillation may optionally be adjusted while the amount of evaporation of ethanol is checked. After distillation, the concentration of ethanol is once again checked, and when it is found to be still not greater than 80% by volume, another distillation is carried out. When the concentration of ethanol is found to be 80% by volume or greater, the ethanol may be passed on to the next recycling step.

### (Recycling-of-Ethanol Step) (when the concentration of ethanol is found to be 80% by volume or greater)

This step will be explained mainly with reference to the ethanol recycling step of Figure 6.

The ethanol found to have a concentration of 80% by volume or greater may immediately be recycled in common step 2 or pectin step 2 where ethanol is used (Figure 6).

It is here to be noted that the "ethanol step" described herein includes both the ethanol regeneration treatment and the recycling of ethanol.

### (Use of ethanol in mixed form: Figures 6 and 7)

The ethanol found to have a concentration of 80% by volume or greater may be used in mixed form in common step 2 and pectin step 2 irrespective of whether it is fresh ethanol A, regenerated ethanol B or recyclable ethanol C. Note here that the concentration of ethanol may be checked by such various check methods as described in the above paragraphs of "(Ethanol step)(Figures 6 and 7)","(Regeneration of Ethanol)" and "(Checking of Concentration of Ethanol)" in addition to the one shown in Figure 6, although all available ones are not shown.

### Example 2

### (Water treatment in mixed form: Figures 3, 4 and 5)

Another example of the invention is now explained.

In Example 2, whole apples and apple juice residues in mixed form are fed to common step 1 and the following steps. In the present disclosure, this is called the water treatment in mixed form. Both the whole apples and the apple juice residues are in themselves derived from individual apple fruits; both are not excluded from each other with high treatment efficiency.

### (Common Step 1: Figures 3-5 and 7)

One hundred twenty (120) liters of water were added to 30 kg of juice residues occurring from apple juice production for a 30-minute stirring in a hand mixer. After stirring, the solution was filtered through a sieve having a pore diameter of 180 µm for separation of water from the juice residues. The juice residues were dried at 80°C for 16 hours to obtain 3.3 kg of dried juice residues.

### (Common Step 2: Figures 3-5 and 7)

Sixteen (16) liters of ethanol were added to 1 kg of dried juice residues for a 2-hour stirring in a stirrer. After stirring, the solution was filtered through a sieve having a pore diameter of 180 µm to obtain 13.3 liters of an ethanol treatment solution. A solid matter remaining on the sieve was dried to obtain 0.88 kg of dried ethanol treatment residues.

### (Ceramide Step: Figures 3, 5 and 7)

The ethanol treatment solution was concentrated in a rotary evaporator to obtain a concentrate and 0.75 liter of recovered ethanol (1). This concentrate was refined to obtain 6.3 g of a ceramide-containing fraction.

### (Pectin Step 1: Figures 4, 5 and 7)

Ninety (90) liters of 0.05N hydrochloric acid were added to 1 kg of the dried ethanol treatment residues, and they were stirred with the application of heat. After the temperature of the solution went up to 80°C, a 30-minute stirring was carried out with that temperature maintained. After stirring, the solution was filtered through a sieve having a pore diameter of 180 µm to obtain 64.8 liters of the hydrochloric acid treatment solution.

### (Pectin Step 2: Figures 4, 5 and 7)

One (1) liter of the hydrochloric acid extraction solution was concentrated under reduced pressure in a rotary evaporator to obtain 0.17 liter of a concentrate. This concentrate was stirred with 0.34 liter of ethanol added to it, resulting in a precipitate that was then separated off through a sieve having a pore diameter of 180 µm. Here 0.38 liter of recovered ethanol (2) was obtained together with the precipitate. The precipitate was stirred with 0.3 liter of ethanol added to it for about 5 minutes, and again separated off through a sieve having a pore diameter of 180 µm. This precipitate was freeze-dried to obtain 2.3 g of pectin.

### (Ethanol Treatment Step: Figures 6 and 7)

Recovered ethanol (2) was found to have an ethanol concentration of 72% by volume as measured by a mechanical oscillator densitometer. This ethanol (2), because of having an ethanol concentration of less than 80% by volume, was regenerated in an ethanol recovery apparatus (GAlA6M-18 available from KNK). Two hundred ten (210) liters of recovered ethanol (2) were loaded in the ethanol recovery apparatus that was then put in operation at a heating temperature of 130°C for a heating time of 8 hours under atmospheric pressure. As a consequence, 6.3 liters of regenerated ethanol were obtained. The regenerated ethanol was found to have an ethanol concentration of 84% by volume as measured by a mechanical oscillator densitometer.

### Example 3

### (Water Treatment in Separated Form: Figures 3, 4 and 5)

In yet another example of the invention, water treatment is applied to whole apples after crushing treatment or mashing treatment (in common step 1); however, the crushed or mashed whole apples may be water treated apart from the water treatment of apple juice residues (water treatment in separated form). The intention of this water treatment in separated form is to water-treat whole apples comprising individual apples having relatively high proportions of water-soluble ingredients and apple juice residues with the proportions of water-soluble ingredients made relatively low as a result of the juice-extraction step in mutually different units or groups. As a consequence, the water treatment can be carried out more rapidly, more efficiently than the water treatment where both are water treated in the common step as in Example 2.

### Example 4

### (Figures 6 and 7)

A further example of the invention is now explained.

The invention may be embodied in the form of a method of using ethanol in a method of extracting ceramide and/or pectin from whole apples and/or apple juice residues, characterized in that the ethanol used comprises at least two of fresh ethanol (A), regenerated ethanol (B) and recyclable ethanol (C).

In the invention here, the fresh ethanol comprises unused ethanol (A) (Figure 7).

### (Explanation of an ethanol regeneration treatment/recycle)

An ethanol regeneration treatment flow is now explained with reference to Figure 6.

The ethanol recovered from the ceramide step, and the ethanol recovered from the pectin step is checked for ethanol concentration. After checking, the ethanol found to have a concentration of 80% by volume or greater is immediately passed as recyclable ethanol on to common step 2 and/or the pectin step (recyclable ethanol). The ethanol found to have a concentration of less than 80% by volume as a result of concentration checking, on the other hand, is regenerated as regenerated ethanol (B) (having a concentration of 80% by volume or greater) that is then passed on to common step 2 and/or the pectin step (regenerated ethanol). After the regeneration treatment, the regenerated ethanol found to have a concentration of 80% by volume or greater, too, provides recyclable ethanol (C) (Figures 6 and 7).

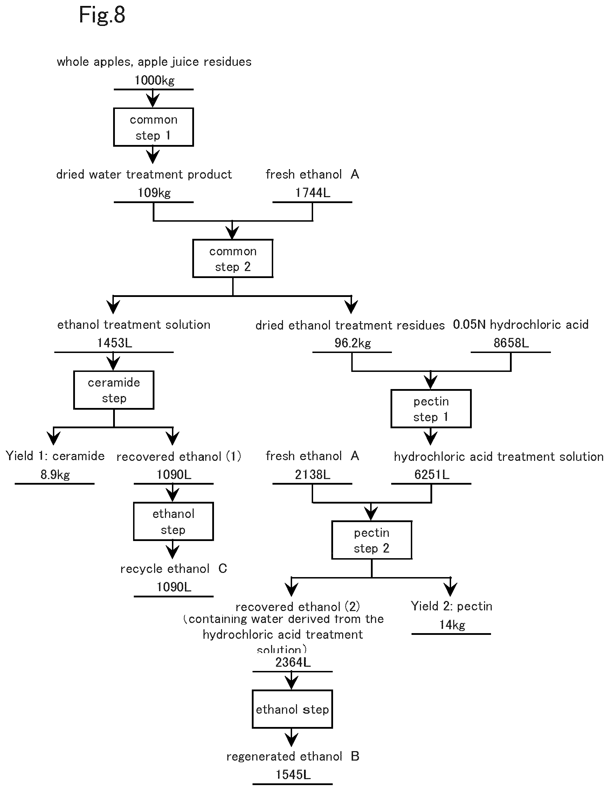

### (Material Balance: Figure 8)

The invention here is explained with reference to the material balance of Figure 8 upon extraction of ceramide and pectin.

A total of 1,000 kg of "whole apples" and "juice residues" were passed on to common step 1 to obtain 109 kg of dried water-treatment product. This, with 1,744 liters of fresh ethanol added to it, was fed to common step 2.

As a result, there were 1,453 liters of ethanol treatment solution and 96.2 kg of dried ethanol treatment residues obtained respectively. As 1,453 liters of the ethanol treatment solution were passed on to the ceramide step, it yielded 8.9 kg of ceramide and 1,090 liters of recovered ethanol (1). Recovered ethanol (1) found to have an ethanol concentration of 80% by volume or greater as a result of checking could be used as recyclable ethanol. On the other hand, 96.2 kg of the dried ethanol treatment residues, with the addition of 8,658 liters of 0.05N hydrochloric acid, were passed on to pectin step 1, resulting in recovery of 6,251 liters of hydrochloric acid treatment solution. As 6,251 liters of this hydrochloric acid treatment solution, with 2,138 liters of fresh ethanol (A) added to it, were fed to pectin step 2, it yielded 14 kg of pectin and 2,364 liters of recovered ethanol (2). Recovered ethanol (2), because of being a mixture of water derived from the hydrochloric acid treatment solution with the fresh ethanol, was obtained in an amount larger than that of the added fresh ethanol. As 2,364 liters of recovered ethanol (2) were passed on to the ethanol recycling flow of Figure 6, it yielded 1,545 liters of regenerated ethanol. While the checking of ethanol concentration is carried out with similar treatment processes in mind, an additional ethanol recovery may be carried out by taking hold of an ethanol concentration of 80% by volume or greater.

### APPLICABILITY TO THE INDUSTRY

### (Applicability-to-the-Industry 1)

The present invention described herein provides a method of extracting useful ingredients: ceramide and pectin from "whole apples" and/or apple juice residues. The present invention also provides a method of extracting ceramide and pectin from apple juice residues in a continuous or sequential way. In this conjunction, the present invention enables a resource used as ethanol to be regenerated or recycled without throwing away it. Thus, the present invention is useful to the industry.

The present invention is also applied not only to apple juice residues but also to "whole apples" as can be appreciated from the aforesaid definition. In other words, it is possible to extract ceramide and pectin from all as-harvested apples retaining apple form like fallen apples, unripe apples, and apples excluded in a sorting process. This contributes more to improvements in the storability and distribution capability of as-harvested apples in varying forms. It is in turn possible to create jobs in related industrial sections and businesses and expand industrial infrastructure as well.

### (Applicability-to-the-Industry 2)

Generally, fallen apples, unripe apples or the like in the form of "whole apples" are recovered from farmers (a primary industry) while "whole apples" occur in the process of being eaten raw or sorting (distribution) at sorting areas. On the other hand, apple juice residues or apple processing byproducts including peels or cores such as those occurring in the process of cutting apples occur in various processing steps at juice-extraction plants, juice production plants, processing plants or the like, and occur in various industrial sections.

In the present invention, the "apple processing byproducts", too, may be used as "recyclable resources" for apple juice residues.

Further, these "whole apples" and the "apple juice residues" may be used in mixed form. Regardless of kinds, the "whole apples" that are not used so far in the respective stages of primary farmers for apples, secondary distributers, tertiary juice-extraction plants or the like and the "apple juice residues" including "apple processing byproducts" may be used alone or in combination for facilitated utilization. Thus, the applicability of the invention to the industry is very promising.

## Claims

1. A method of extracting ceramide from whole apples and/or apple juice residues, **characterized by** comprising:
a common step 1 including water treatment for adding water to the whole apples and/or apple juice residues for stirring, and drying treatment for drying a product obtained by said water treatment to obtain a dried water-treatment product,
a common step 2 including ethanol treatment for adding ethanol to the dried water-treatment product obtained in said common step 1 for stirring, and drying treatment for drying treated residues obtained by said ethanol treatment to obtain an ethanol treatment solution and/or dried ethanol treatment residues, and
a ceramide step of recovering ethanol from the ethanol treatment solution obtained in said common step 2 while concentrating the ethanol treatment solution, and refining the concentrated ethanol treatment solution to obtain ceramide.

2. A method of extracting ceramide as recited in claim 1, wherein ethanol removed in a process of concentrating the ethanol treatment solution in said ceramide step is recovered and recycled.

3. A method of extracting ceramide as recited in claim 2, further includes a step of regenerating ethanol by bringing up a concentration thereof after the step of recovering said ethanol.

4. A method of extracting pectin from whole apples and/or apple juice residues, comprising:
the common step 1 as recited in claim 1,
the common step 2 as recited in claim 1,
a pectin step 1 of adding hydrochloric acid to the dried ethanol treatment residues obtained in said common step 2 for stirring and filtration thereby separating off a hydrochloric acid treatment solution, and
a pectin step 2 of concentrating the hydrochloric acid treatment solution obtained in said pectin step 1, adding ethanol to said hydrochloric acid treatment solution for stirring and filtration thereby obtaining pectin, and drying said pectin.

5. A method of extracting pectin as recited in claim 4, wherein the ethanol used in said pectin step 2 is recovered and recycled.

6. A method of extracting pectin as recited in claim 5, further includes a step of regenerating ethanol by bringing up a concentration thereof after the step of recovering said ethanol.

7. A method of extracting ceramide and pectin from whole apples and/or apple juice residues, comprising:
carrying out a pectin step 1 of adding hydrochloric acid to treatment residues obtained in said common step 2 for stirring and filtration thereby separating off a hydrochloric acid treatment solution after the common step 1 and common step 2 as recited in claim 1 and independently of said ceramide step, and carrying out a pectin step 2 of concentrating a hydrochloric acid treatment solution obtained in said pectin step 1, adding ethanol to said hydrochloric acid treatment solution for stirring and filtration thereby obtaining pectin, and drying said pectin.

8. A method of extracting ceramide and pectin as recited in claim 7, wherein said ceramide step as well as said pectin steps 1 and 2 are concurrently carried out.

9. A method of extracting ceramide and pectin as recited in claim 7 or 8, wherein the whole apples and apple juice residues are mixed together for feeding to said common step 1.

10. A method of extracting ceramide and/or pectin as recited in any one of claims 1 to 9, wherein the ethanol used comprises a mixture of at least two of fresh ethanol, recycled ethanol, and ethanol regenerated by bringing up concentration.

## Patentansprüche

1. Verfahren zum Extrahieren von Ceramid aus ganzen Äpfeln und/oder Apfelsaftrückstand,
**dadurch gekennzeichnet,**
**dass** es folgendes umfasst:
einen gemeinsamen Schritt 1, welcher eine Wasserbehandlung zum Hinzufügen von Wasser zu den ganzen Äpfeln und /oder dem Apfelsaftrückstand zum Zwecke des Umrührens sowie eine Trocknungsbehandlung zum Trocknen eines durch die Wasserbehandlung erhaltenen Produktes beinhaltet, um ein getrocknetes, wasserbehandeltes Produkt zu erhalten,
einen gemeinsamen Schritt 2, der eine Ethanolbehandlung zum Hinzufügen von Ethanol zum in dem gemeinsamen Schritt 1 erhaltenen getrockneten, wasserbehandelten Produkt zum Zwecke des Umrührens sowie eine Trocknungsbehandlung zum Trocknen der behandelten,
durch die Ethanolbehandlung erhaltenen Rückstände beinhaltet, um eine mit Ethanol behandelte Lösung und/oder getrockneten Ethanolbehandlungsrückstand zu erhalten, und
einen Ceramid-Schritt, bei welchem das Ethanol aus der mit Ethanol behandelten Lösung, die in dem gemeinsamen Schritt 2 erhalten wurde, zurückgewonnen wird, während die mit Ethanol behandelte Lösung aufkonzentriert wird, und bei welchem die aufkonzentrierte, mit Ethanol behandelte Lösung gereinigt wird, um das Ceramid zu erhalten.

2. Verfahren zum Extrahieren von Ceramid nach Anspruch 1, bei welchem das Ethanol, welches in einem Prozess der Aufkonzentrierung der mit Ethanol behandelten Lösung im Ceramid-Schritt entfernt wurde, wiedergewonnen und recycelt wird.

3. Verfahren zum Extrahieren von Ceramid nach Anspruch 2, welches weiter einen Schritt enthält, bei welchem das Ethanol regeneriert wird, indem dessen Konzentration nach dem Schritt der Wiedergewinnung von Ethanol erhöht wird.

4. Verfahren zum Extrahieren von Pectin aus ganzen Äpfeln und/oder Apfelsaftrückstand, umfassend:
den gemeinsamen Schritt 1 aus Anspruch 1,
den gemeinsamen Schritt 2 aus Anspruch 1,
einen Pectin-Schritt 1, in welchem den getrockneten,
mit Ethanol behandelten Rückständen, die im gemeinsamen Schritt 2 erhalten wurden, zum Zwecke des Umrührens und
der Filtration Salzsäure hinzugefügt wird, wodurch eine mit Salzsäure behandelte Lösung absepariert wird, und
einen Pectin-Schritt 2, in welchem die in dem Pectin-Schritt 1 erhaltene, mit Salzsäure behandelte Lösung aufkonzentriert wird, in welchem Ethanol der mit Salzsäure behandelten Lösung zum Zwecke des Umrührens und der Filtration hinzugefügt wird, um Pectin zu erhalten, und in welchem das Pectin getrocknet wird.

5. Verfahren zum Extrahieren von Pectin nach Anspruch 4, bei welchem das in dem Pectin-Schritt 2 verwendete Ethanol zurückgewonnen und recycelt wird.

6. Verfahren zum Extrahieren von Pectin nach Anspruch 5, welches weiter einen Schritt enthält, bei welchem Ethanol regeneriert wird, indem dessen Konzentration nach dem Schritt der Wiedergewinnung von Ethanol erhöht wird.

7. Verfahren zum Extrahieren von Ceramid und Pectin aus ganzen Äpfeln und/oder Apfelsaftrückstand, umfassend:
Durchführen eines Pectin-Schritts 1, in welchem zu den Behandlungsrückständen, die in dem gemeinsamen Schritt 2 erhalten wurden, zum Zwecke des Umrührens und der Filtration Salzsäure hinzugefügt wird, wodurch eine mit Salzsäure behandelte Lösung nach dem gemeinsamen Schritt 1 und dem gemeinsamen Schritt 2 aus Anspruch 1 und unabhängig vom Ceramid-Schritt absepariert wird,
sowie Durchführen eines Pectin-Schritts 2, in welchem eine mit Salzsäure behandelte Lösung, die im Pectin-Schritt 1 erhalten wurde, aufkonzentriert wird, in welchem Ethanol zur mit Salzsäure behandelten Lösung zum Zwecke des Umrührens und der Filtration hinzugefügt wird, um dadurch Pectin zu erhalten, und in welchem das Pectin getrocknet wird.

8. Verfahren zum Extrahieren von Ceramid und Pectin nach Anspruch 7,
bei welchem der Ceramid-Schritt und die Pectin-Schritte 1 und 2 gleichzeitig ausgeführt werden.

9. Verfahren zum Extrahieren von Ceramid und Pectin nach Anspruch 7 oder 8,
bei welchem die ganzen Äpfel oder der Apfelsaftrückstand miteinander gemischt werden, um sie dem gemeinsamen Schritt 1 zuzuführen.

10. Verfahren zum Extrahieren von Ceramid und/oder Pectin nach einem der Ansprüche 1 - 9,
bei welchem das verwendete Ethanol eine Mischung von wenigstens zwei der folgenden Komponenten umfasst:
Ethanol, recyceltes Ethanol, Ethanol, welches durch Aufkonzentrieren regeneriert wurde.

## Revendications

1. - Procédé d'extraction de céramide à partir de pommes entières et/ou de résidus de jus de pomme, **caractérisé par** le fait comprend :
une étape commune 1, comprenant un traitement à l'eau pour ajouter de l'eau aux pommes entières et/ou aux résidus de jus de pomme en vue d'une agitation, et un traitement de séchage pour sécher un produit obtenu par ledit traitement à l'eau afin d'obtenir un produit de traitement à l'eau séché,
une étape commune 2, comprenant un traitement à l'éthanol pour ajouter de l'éthanol au produit de traitement à l'eau séché obtenu dans ladite étape commune 1 en vue d'une agitation, et un traitement de séchage pour sécher des résidus traités obtenus par ledit traitement à l'éthanol afin d'obtenir une solution de traitement à l'éthanol et/ou des résidus de traitement à l'éthanol séchés, et
une étape de céramide consistant à récupérer l'éthanol à partir de la solution de traitement à l'éthanol obtenue dans ladite étape commune 2 tout en concentrant la solution de traitement à l'éthanol, et raffiner la solution de traitement à l'éthanol concentrée pour obtenir du céramide.

2. - Procédé d'extraction de céramide tel que décrit à la revendication 1, dans lequel l'éthanol retiré dans un processus de concentration de la solution de traitement à l'éthanol dans ladite étape de céramide est récupéré et recyclé.

3. - Procédé d'extraction de céramide tel que décrit à la revendication 2, comprenant en outre une étape consistant à régénérer l'éthanol en élevant une concentration de celui-ci après l'étape de récupération dudit éthanol.

4. - Procédé d'extraction de pectine à partir de pommes entières et/ou de résidus de jus de pomme, comprenant :
l'étape commune 1 telle que décrite à la revendication 1,
l'étape commune 2 telle que décrite à la revendication 1,
une étape de pectine 1 consistant à ajouter de l'acide chlorhydrique aux résidus de traitement à l'éthanol séchés obtenus dans ladite étape commune 2 en vue d'une agitation et d'une filtration, séparant ainsi une solution de traitement à l'acide chlorhydrique, et
une étape de pectine 2 consistant à concentrer la solution de traitement à l'acide chlorhydrique obtenue dans ladite étape de pectine 1, ajouter de l'éthanol à ladite solution de traitement à l'acide chlorhydrique en vue d'une agitation et d'une filtration, obtenant ainsi de la pectine, et sécher ladite pectine.

5. - Procédé d'extraction de pectine tel que décrit à la revendication 4, dans lequel l'éthanol utilisé dans ladite étape de pectine 2 est récupéré et recyclé.

6. - Procédé d'extraction de pectine tel que décrit à la revendication 5, comprenant en outre une étape consistant à régénérer l'éthanol en élevant une concentration de celui-ci après l'étape de récupération dudit éthanol.

7. - Procédé d'extraction de céramide et de pectine à partir de pommes entières et/ou de résidus de jus de pomme, consistant à :
réaliser une étape de pectine 1 consistant à ajouter de l'acide chlorhydrique à des résidus de traitement obtenus dans ladite étape commune 2 en vue d'une agitation et d'une filtration, séparant ainsi une solution de traitement à l'acide chlorhydrique après l'étape commune 1 et l'étape commune 2 selon la revendication 1 et indépendamment de ladite étape de céramide, et réaliser une étape de pectine 2 consistant à concentrer une solution de traitement à l'acide chlorhydrique obtenue dans ladite étape de pectine 1, ajouter de l'éthanol à ladite solution de traitement à l'acide chlorhydrique en vue d'une agitation et d'une filtration, obtenant ainsi de la pectine, et sécher ladite pectine.

8. - Procédé d'extraction de céramide et de pectine tel que décrit à la revendication 7, dans lequel ladite étape de céramide ainsi que lesdites étapes de pectine 1 et 2 sont réalisées simultanément.

9. - Procédé d'extraction de céramide et de pectine tel que décrit à la revendication 7 ou 8, dans lequel les pommes entières et les résidus de jus de pomme sont mélangés ensemble en vue d'une fourniture à ladite étape commune 1.

10. - Procédé d'extraction de céramide et/ou de pectine tel que décrit à l'une quelconque des revendications 1 à 9, dans lequel l'éthanol utilisé comprend un mélange d'au moins deux éléments parmi de l'éthanol neuf, de l'éthanol recyclé et de l'éthanol régénéré en élevant la concentration.
